Europäisches Patentamt

European Patent Office

Office européen des brevets

(19)

(11) Numéro de publication : **0 288 362 B1**

(12) **FASCICULE DE BREVET EUROPEEN**

(45) Date de publication du fascicule du brevet :
03.04.91 Bulletin 91/14

(51) Int. Cl.$^5$ : **C07C 11/08, C07C 5/25**

(21) Numéro de dépôt : 88400911.9

(22) Date de dépôt : **15.04.88**

(54) **Procédé d'isomérisation du butène-1 en butènes-2 dans une coupe d'hydrocarbures en c4 contenant du butadiène et des composés sulfurés.**

(30) Priorité : 22.04.87 FR 8705735

(43) Date de publication de la demande :
26.10.88 Bulletin 88/43

(45) Mention de la délivrance du brevet :
03.04.91 Bulletin 91/14

(84) Etats contractants désignés :
AT DE GB IT NL

(56) Documents cités :
US-A- 4 132 745

(73) Titulaire : **INSTITUT FRANCAIS DU PETROLE**
**4, Avenue de Bois-Préau**
**F-92502 Rueil-Malmaison (FR)**

(72) Inventeur : **Boitiaux, Jean-Paul**
**4, avenue des Ursulines**
**F-78300 Poissy (FR)**
Inventeur : **Cosyns, Jean**
**50, route d'Herbeville**
**F-78580 Maule (FR)**

## Description

Les procédés de conversion d'hydrocarbures à haute température tels que, par exemple, le vapocraquage, la viscoréduction, la cokéfaction et le craquage catalytique produisent une grande quantité d'hydrocarbures insaturés de nature oléfinique et, en particulier, des coupes d'hydrocarbures en $C_4$ contenant des oléfines telles que le butène-1, les butènes-2, l'isobutène ainsi que du butadiène.

De telles coupes peuvent être utilisées pour la production d'intermédiaires pétrochimiques ou bien pour la production de carburants.

Par exemple, dans le cas du craquage catalytique, la coupe d'hydrocarbures en $C_4$ produite est utilisée comme charge du procédé d'alkylation dans lequel elle est transformée en essence à haut indice d'octane. Un exemple de composition d'une coupe $C_4$ de craquage catalytique en lit fluide (FCC : Fluid Catalytic Cracking) est donné ci-après.

| Hydrocarbures | % poids |
|---|---|
| Isobutane | 34,5 |
| Isobutène | 15 |
| Butène-1 | 13 |
| Butadiène | 0,5 |
| Butène-2 cis | 10 |
| N-butane | 11 |
| Butènes-2 | 16 |
| Total | 100 |

De telles coupes contiennent, en outre, des traces de composés sulfurés dont la concentration est, en général, comprise entre 5 et 50 ppm comptées en soufre.

Le procédé d'alkylation consiste à alkyler les oléfines telles que butènes et isobutène sur l'isobutane en présence d'acide sulfurique ou d'acide fluorhydrique comme catalyseur. On obtient, ainsi, une coupe à haut indice d'octane (indices d'octane "recherche" et "moteur") particulièrement valorisable comme base de carburant auto.

Cependant, les coupe $C_4$ provenant du FCC ne peuvent être utilisées telles que, car elles contiennent du butadiène. Celui-ci entraîne, en effet, une surconsommation d'acide particulièrement dans l'alkylation sulfurique, par formation de boues acides qu'il faut éliminer. Pour cette raison, on demande donc d'hydrogéner le butadiène de manière à obtenir une teneur résiduelle généralement inférieure à 100 ppm. De plus, il est souvent demandé de transformer le butène-1 en butènes-2 cis et trans. En effet, les alkylats provenant de ces dernières oléfines ont des indices d'octane nettement plus élevés ; ceci est particulièrement observé dans le cas de l'alkylation effectuée en présence d'acide fluorhydrique.

On a, depuis longtemps, proposé d'une part, des procédés d'hydrogénation du butadiène et d'autre part, des procédés d'isomérisation du butène-1 en butènes-2. L'hydrogénation du butadiène est maintenant le plus souvent réalisée à basse température et en phase liquide sur des catalyseurs à base de palladium. Il est recommandé, dans ce type de procédé, d'opérer en absence de composés sulfurés ; ceux-ci, en effet, s'ils sont présents, même à faible concentration, généralement de l'ordre de 10 ppm environ, peuvent inhiber complètement la réaction d'hydrogénation. L'isomérisation du butène-1 en butènes-2 est également réalisée sur des catalyseurs à base de palladium et en présence d'hydrogène. Mais, pour rendre ceux-ci sélectifs, c'est-à-dire pour éviter la formation de butane, on a recommandé de présulfurer le catalyseur de diverses manières (brevet USP n° 4.132.745).

Dans le cas où l'on droit traiter une coupe $C_4$ oléfinique contenant des composés sulfurés et du butadiène afin, non seulement, d'hydrogéner ce dernier, mais également d'isomériser le butène-1 en butènes-2, les procédés mentionnés ci-dessus se révèlent très vite inopérants. Ainsi, par exemple, lorsque les teneurs en soufre sont supérieures à 10 ppm, on constate que la conversion du butadiène devient trop faible, tandis que l'isomérisation du butène-1 en butènes-2 devient négligeable. On peut, éventuellement, arriver à réaliser des deux réactions, mais dans des conditions de température et de pression qui deviennent rapidement non économiques.

Le procédé de l'invention permet de remédier à ces inconvénients. Il consiste à opérer avec deux lits de catalyseurs différents : le premier lit traversé par la charge est un catalyseur d'hydrogénation contenant du palladium et un deuxième métal qui est l'or et/ou le platine. Ces métaux sont déposés sur un support qui de préférence peut être un support d'alumine (inerte ou non) (par "inerte" on désigne une alumine de faible surface spécifique) ou de silice. Le second lit est un catalyseur à base de palladium seul déposé sur un support du même type (alumine ou silice). La charge à traiter doit donc d'abord passer sur le premier catalyseur. La température moyenne dans le réacteur devra être comprise entre 20 et 150°C. On peut s'arranger pour travailler dans chacun des deux lits à différentes températures et pressions. Mais, il est souvent préféré, notamment lorsque les deux lits se trouvent dans le même zone réactionnelle, d'opérer dans les mêmes conditions opératoires dans les deux lits. On préférera alors travailler à température pas trop élevée (inférieure, par exemple, à 100°C) car l'équilibre thermodynamique d'isomérisation du butène-1 en butènes-2 est plus favorable à basse température. Cependant, la présence de composés sulfurés dans la charge impose une température relativement élevée (généralement supérieure à 60°C) pour obtenir une conversion suffisante du butadiène 1-3. Or, grâce aux deux lits de catalyseurs distincts de l'invention, il est possible de travailler dans un domaine de températures acceptables économiquement c'est-à-dire de préférence 60 à 100°C. La pression peut être choisie dans un large domaine par exemple entre 1 et 50 atmosphères et de préférence entre 5 et 30 atmosphères. En général, on préfère que la coupe $C_4$ soit maintenue en majeure partie en phase liquide. Le débit de la charge à traiter est habituellement de 1 litre à 50 litres et de préférence 2 litres à 30 litres (à l'état liquide) par litre de catalyseur et par heure. Le débit d'hydrogène introduit est essentiellement fonction de la teneur en butadiène de la coupe $C_4$.

Un second avantage du procédé selon l'invention est son aptitude à hydrogéner le butadiène et isomériser le butène-1 en présence d'hydrogène sulfuré. En effet, les catalyseurs traditionnels ne pouvant fonctionner en présence de composés sulfurés qu'à des températures suffisamment élevées, il est nécessaire d'augmenter la pression du traitement pour maintenir la coupe en phase liquide. Or, l'hydrogène pur disponible en raffinerie est généralement à trop basse pression, il est donc nécessaire de comprimer cet hydrogène, ce qui le rend très coûteux. La résistance à l'hydrogène sulfuré du système catalytique proposé a, en revanche, le gros avantage de permettre l'utilisation d'hydrogène de purge d'hydrotraitement comme gaz hydrogénant qui contient généralement entre 100 et 10000 ppm volume d'hydrogène sulfuré.

Le premier lit catalytique contenant soit du palladium et de l'or, soit du palladium et du platine peut être préparé de toute manière convenable, par exemple en imprégnant un support minéral adéquat avec une solution aqueuse ou organique de composés des métaux que l'on désire déposer. Ces composés pourront être des sels minéraux ou des complexes organiques tels que, pour les minéraux, les chlorures ou les nitrates et, pour les complexes organiques, les acétylacétonates par exemple. L'imprégnation des deux métaux pourra se faire en une ou deux étapes avec, entre les deux étapes éventuelles, un traitement intermédiaire de calcination et (ou) de réduction. On peut, par exemple, utiliser la technique décrite dans le brevet USP n° 4,490,481.

Après la ou les étapes d'imprégnation, le catalyseur peut être calciné à l'air, puis réduit de toute manière appropriée, de façon à l'amener dans sa forme active. Le support minéral devra être inerte pour éviter les réactions de polymérisation du butadiène ou des butènes ; ce pourra être, par exemple, comme indiqué plus haut, une alumine d'une surface spécifique de préférence inférieure à 100 m²/g ou de la silice.

Le catalyseur du deuxième lit renferme du palladium déposé de toute manière appropriée sur un support d'alumine ou de silice. Bien que cela ne soit pas limitatif, on préfère imprégner le support avec une solution aqueuse de chlorure de palladium. Ensuite, le catalyseur est généralement calciné à l'air, puis réduit de toute manière appropriée pour l'amener dans sa forme active.

D'une manière générale, la proportion en volume du premier catalyseur par rapport à la somme des volumes des deux lots catalytiques est comprise entre 10 et 70% et de préférence entre 20 et 50%.

Le pourcentage pondéral en palladium par rapport au catalyseur total est compris entre 0,05 et 1%, de préférence entre 0,1 et 0,5% et plus particulièrement entre 0,15 et 0,35%. Les concentrations pondérales en or ou platine sont comprises entre 0,01 et 0,2%, de préférence entre 0,03 et 0,15% et plus particulièrement entre 0,04% et 0,12%.

Les exemples suivants, donnés à titre non limitatif, illustrent la présente invention.

Les métaux, palladium, or ou platine, utilisés ici sont présents sur le support sous une forme métallique ou sous forme de composés métalliques.

Exemple 1. (comparatif)

Dans cet exemple, on choisit un support d'alumine se présentant sous forme de billes de 2 à 4 mm de diamètre, d'une surface spécifique de 70 m²/g, d'un volume poreux total de 0,6 cm³/g et ayant un diamètre moyen de pores de 200 angströms ($200 \times 10^{-10}$ mètres). Avec ce support, on prépare, en utilisant la méthode du brevet USP n° 4,490,481, deux catalyseurs bimétalliques dont les teneurs en métaux sont les suivantes :

* Catalyseur 1 :    0,2% poids Pd
                      0,08% poids Au

*Catalyseur 2 :    0,02% poids Pd
                      0,08% poids Pt

Sur le même support, on prépare également deux catalyseurs ne contenant que du palladium.

Le catalyseur 3 est préparé par imprégnation à sec à l'aide d'une solution aqueuse de nitrate de palladium; il est ensuite séché et calciné sous air à 450°C pendant deux heures. Le catalyseur 4 est préparé de la même manière, mais en utilisant le chlorure de palladium.

La teneur en métal palladium de ces deux catalyseurs est de 0,3% poids.

On traite une coupe $C_4$, provenant d'un craquage catalytique en lit fluide, dont la composition pondérale est la suivante :

|  | % poids |
|---|---|
| Total hydrocarbures en $C_3$ | 0,15 |
| Isobutane | 25,13 |
| N-butane | 9,67 |
| Butène-1 | 14,80 |
| Isobutène | 17,92 |
| Butène-2 trans | 17,31 |
| Butène-2 cis | 12,53 |
| Butadiène 1-3 | 0,52 |
| Total hydrocarbures en $C_5$ | 1,97 |
| Soufre (ppm) | 20 |

Les catalyseurs préparés ci-dessus sont essayés successivement dans une installation d'hydrogénation comportant un réacteur tubulaire dans lequel on place le catalyseur. Ces essais ne sont donc pas conformes à l'invention.

Avant chaque essai d'hydrogénation, chaque catalyseur est réduit sous un courant d'hydrogène à 150°C pendant 2 heures.

Les conditions opératoires du test d'hydrogénation sont les suivantes :
* Pression : 25 bar
* Température : 80°C
* VVH : 15 litres par litre de catalyseur et par heure
* $H_2$/butadiène : 2,2 moles/mole

Pour les quatre catalyseurs, le rendement pondéral en produit par rapport à la charge introduite est pratiquement égal à 100%. On constate, sur les quatre catalyseurs, une hydrogénation plus ou moins poussée du butadiène, ainsi qu'une certaine isomérisation du butène-1 en butènes-2. Dans tous les cas, la proportion d'oléfines hydrogénées en paraffines est négligeable. Les résultats comparés sont résumés dans le tableau 1.

Tableau 1.

| Catalyseurs | Butadiène résiduel ppm | Conversion butadiène % | Butène-1 résiduel % | Conversion butène-1 % |
|---|---|---|---|---|
| 1 (Pd Au) | 10 | 99,8 | 8,73 | 41 |
| 2 (Pd Pt) | 20 | 99,6 | 9,03 | 39 |
| 3 (Pd ex nitrate) | 260 | 95 | 8,14 | 45 |
| 4 (Pd ex chlorure) | 280 | 94,6 | 7,10 | 52 |

On constate que certains catalyseurs convertissent de façon importante le butadiène, mais aucun ne permet de transformer substantiellement le butène-1. En effet, si les catalyseurs se montraient suffisamment actifs pour cette isomérisation, on devrait trouver des concentrations résiduelles en butène-1 nettement plus proches de la concentration à l'équilibre thermodynamique pour la température choisie, à savoir : 2,23%, la conversion théorique obtenue avec la teneur en butène-1 de 14,80 de la charge considérée est donc de : 14,80 − 2,23/14.80 × 100 = 84,9%.

Exemple 2 (selon l'invention).

Dans cet exemple, on traite la même charge que celle de l'exemple 1 et dans les mêmes conditions.

En revanche, le réacteur est rempli avec deux lits de catalyseurs ; le premier lit est le catalyseur n° 1 (palladium-or) et représente 30% du volume total utilisé de catalyseur ; le second lit est le catalyseur n° 3 (palladium-nitrate) qui représente le complément, c'est-à-dire 70% en volume. Les résultats sont résumés dans le tableau 2.

Tableau 2.

| Butadiène résiduel (ppm) | : | 60 |
|---|---|---|
| Conversion butadiène % | : | 98,8 |
| Butène-1 résiduel % | : | 5,2 |
| Conversion butène-1 % | : | 64,9 |

Par rapport aux résultats de l'exemple 1, on voit, d'une part, que l'on obtient aisément une teneur résiduelle en butadiène inférieure à 100 ppm et que, d'autre part, le butène-1 subit une transformation beaucoup plus poussée, avec une conversion très convenable du butadiène.

Exemple 3 (selon l'invention).

La charge traitée et les conditions opératoires sont les mêmes que celles de l'exemple 1.

Le réacteur est rempli de la même manière que dans l'exemple 2, c'est-à-dire avec un premier lit représentant 30% du volume total utilisé de catalyseur ; le second représentant le complément, soit 70% en volume. On a effectué deux essais avec respectivement les catalyseurs 1 (Pd-Au) et 4 (Pd-chlorure) d'une part et les catalyseurs 2 (Pd-Pt) et 3 (Pd-nitrate) d'autre part. Les résultats obtenus sont résumés dans le tableau 3.

Tableau 3.

| Catalyseurs | | (1) + (4) | (2) + (3) |
|---|---|---|---|
| Butadiène résiduel (ppm) | : | 55 | 80 |
| Conversion butadiène % | : | 98,9 | 98,4 |
| Butène-1 résiduel % | : | 4,2 | 5,3 |
| Conversion butène-1 % | : | 71,6 | 64 |

Exemple 4 (selon l'invention).

La charge traitée et les conditions opératoires sont celles de l'exemple 1. Le réacteur est rempli de la même manière que dans l'exemple 3 ; le catalyseur de tête étant, ici, la préparation n° 2 (Pd Pt) et le catalyseur de queue étant le catalyseur au palladium imprégné sous forme chlorure (préparation n° 4).

Les résultats obtenus sont résumés dans le tableau 4.

6

Tableau 4.

| Butadiène résiduel (ppm) | : | 70 |
|---|---|---|
| Conversion butadiène % | : | 98,7 |
| Butène-1 résiduel % | : | 4,5 |
| Conversion butène-1 % | : | 69,6 |

Exemple 5. (Selon l'invention)

La charge traitée est le même que dans l'exemple 1. Le réacteur est rempli avec deux lits de catalyseurs, le premier lit est le catalyseur n° 1 (palladium-or) et représente 30% du volume total utilisé de catalyseur ; le second lit est le catalyseur n° 4 (palladium-chlorure) qui représente le complément, c'est-à-dire 70% en volume. Les conditions opératoires qui diffèrent des exemples précédents sont les suivantes :

* Pression : 25 bars
* Température : 90°C
* VVH : 10 litres par litre de catalyseur et par heure
* $H_2$/butadiène : 2,2 moles/mole

Les résultats obtenus sont présentés dans le tableau 5.

Tableau 5.

| Butadiène résiduel | : | non détectable |
|---|---|---|
| Conversion butadiène % | : | 100 |
| Butène-1 résiduel % | : | 2,7 |
| Conversion butène-1 | : | 81,7 % |

Exemple 6. (Comparatif)

Dans cet exemple, on traite toujours la même charge et dans les mêmes conditions opératoires que celles de l'exemple 1. En revanche, la disposition des deux catalyseurs est modifiée : le catalyseur placé en premier est celui de la préparation n° 4 (ex chlorure de palladium) et représente 30% du volume total, et le second est la préparation n° 1 (palladium-or). Les résultats obtenus sont résumés dans le tableau 6.

## Tableau 6.

| Butadiène résiduel (ppm) | : | 40 |
|---|---|---|
| Conversion butadiène % | : | 99,2 |
| Butène-1 résiduel % | : | 8,3 |
| Conversion butène-1 % | : | 43,9 |

On voit ici que l'ordre de passage de la charge sur les deux catalyseurs est important. Ainsi, la conversion du butène-1 est très nettement inférieure à celles obtenues dans les exemples réalisés selon l'invention.

Exemple 7 (comparatif et selon l'invention).

On traite ici la même charge que dans l'exemple 1. Mais, l'hydrogène utilisé est impur et contient 1000 ppm molaire d'$H_2S$.

Les conditions opératoires choisies sont celles de l'exemple 5, c'est-à-dire :
* Pression : 25 bars
* Température : 90°C
* VVH : 10
* $H_2$/butadiène : 2,2 moles/mole

On réalise 4 expériences : l'une selon l'invention, dans laquelle le premier catalyseur est (comme dans l'exemple 5) la préparation n° 1 (palladium-or) et représente 30% du volume total, le deuxième catalyseur étant constitué par la préparation n° 4 (Pd-chlorure).

Les autres expériences, réalisées à titre comparatif, sont réalisées respectivement avec le catalyseur n° 1 seul, le catalyseur n° 4 seul et un lit mixte constitué dans l'ordre de 70% de catalyseur n° 4 et 30% de catalyseur n° 1.

Les résultats sont présentés dans le tableau 7.

On constate, ici, que seul le système catalytique de l'invention permet de travailler en présence d'$H_2S$ et de convertir quasi totalement le butadiène et, substantiellement, le butène-1.

Les résultats ne sont cependant pas aussi bons que dans l'exemple 5 mais ils permettent toutefois de comprendre qu'il devient possible, aujourd'hui, d'utiliser avantageusement, en appliquant l'invention, non pas de l'hydrogène pur mais coûteux, mais de l'hydrogène de purge d'hydrotraitement.

Tableau 7.

| | Cata n° 1 (30 %) Cata n° 4 (70 %) | Cata n° 1 seul | Cata n° 4 seul | Cata n° 4 (70 %) Cata n° 1 (30 %) |
|---|---|---|---|---|
| Butadiène résiduel (ppm) | ⩽10 | ⩽10 | 4400 | 250 |
| Conversion butadiène (%) | 100 | 100 | 12 | 95 |
| Butène-1 résiduel (%) | 3,4 | 6,5 | 14 | 11 |
| Conversion butène-1 (%) | 78 | 56 | 5 | 28 |

## Revendications

1. Procédé d'isomérisation du butène-1 en butènes-2 dans une coupe d'hydrocarbures en $C_4$ contenant du butadiène et des composés sulfurés, avec hydrogénation du butadiène, le procédé étant caractérisé en ce que :

(a) on envoie la dite coupe à travers un premier lit d'un catalyseur qui renferme du palladium et au moins un deuxième métal qui est l'or et/ou le platine, ces métaux étant déposés sur un support, en présence d'hydrogène, à une température comprise entre 20 et 150°C, sous une pression comprise entre 1 et 50 atmosphères,

(b) on envoie au moins la majeure partie de l'effluent du premier lit de catalyseur à travers un deuxième lit de catalyseur renfermant du palladium déposé sur de l'alumine ou de la silice, en présence d'hydrogène, à une température comprise entre 20 et 150°C sous une pression comprise entre 1 et 50 atmosphères.

2. Procédé, selon la revendication 1, dans lequel les deux lits de catalyseur sont placés dans une même zone de réaction, la température de la zone de réaction étant comprise entre 60 et 100°C, la pression étant comprise entre 5 et 30 atmosphères.

3. Procédé, selon la revendication 2, dans lequel le palladium du deuxième lit de catalyseur a été introduit sur le support sous la forme d'un nitrate.

4. Procédé, selon la revendication 2, dans lequel le palladium du deuxième lit de catalyseur a été introduit sur le support sous la forme d'un chlorure.

5. Procédé, selon l'une des revendications 1 à 4, dans lequel le support des catalyseurs des deux lits est une alumine de surface spécifique inférieure à 100 m²/g.

6. Procédé, selon la revendication 2, dans lequel la quantité de catalyseur du premier lit représente, en volume, 10 à 70% de la totalité des volumes des deux lits de catalyseurs.

7. Procédé, selon la revendication 4, dans lequel des métaux du premier lit sont le palladium et l'or.

8. Procédé, selon la revendication 4, dans lequel les métaux du premier lit sont le palladium et le platine.

9. Procédé, selon l'une des revendications 1 à 8, dans lequel l'hydrogéne utilisé sur chacun des deux lits de catalyseur renferme 100 à 10000 ppm d'$H_2S$.

10. Procédé selon l'une des revendications 1 à 9, dans lequel la coupe d'hydrocarbures renferme entre 5 et 50 ppm de soufre.

## Ansprüche

1. Verfahren zur Isomerisation von Buten-1 zu Butenen-2 in einem $C_4$-Kohlenwasserstoffschnitt der Butadien und schwefelhaltige Verbindungen enthält, mit Hydrierung des Butadiens, dadurch gekennzeichnet, daß man

a) den genannten Schnitt über ein erstes Bett eines Katalysators der Palladium und zumindest ein zweites Metall, nämlich Gold und/oder Platin enthält, wobei diese Metalle auf einem Träger angeordnet sind, in Anwesenheit von Wasserstoff bei einer Temperatur zwischen 20 und 150°C und unter einem Druck zwischen 1 und 50 Atmosphären passieren läßt und daß man,

b) zumindest dem größeren Teil des Effluents des ersten Katalysatorbetts über ein zweites Katalysatorbett, das Palladium niedergeschlagen auf Aluminiumoxid oder auf Siliciumdioxid enthält, in Anwesenheit von Wasserstoff bei einer Temperatur zwischen 20 und 150°C sowie unter einem Druck zwischen 1 und 50 Atmosphären passieren läßt.

2. Verfahren nach Anspruch 1, dadurch gekennzeichnet, daß die beiden Katalysatorbetten in der gleichen Reaktionszone angeordnet sind, wobei die Temperatur der Reaktionszone zwischen 60 und 100°C beträgt und der Druck zwischen 5 und 30 Atmosphären liegt.

3. Verfahren nach Anspruch 2, dadurch gekennzeichnet, daß das Palladium des zweiten Katalysatorbetts in Form des Nitrats in den Träger eingeführt wird.

4. Verfahren nach Anspruch 2, dadurch gekennzeichnet. daß das Palladium des zweiten Katalysatorbetts in Form des Chlorids in den Träger eingeführt wird.

5. Verfahren nach mindestens einem der Ansprüche 1 bis 4, dadurch gekennzeichnet, daß der Träger der Katalysatoren der beiden Betten aus Aluminiumoxid mit einer spezifischen Oberfläche unterhalb von 100 $m^2/g$ besteht.

6. Verfahren nach Anspruch 2, dadurch gekennzeichnet, daß die Menge an Katalysator des ersten Betts 10 bis 70 Vol.-% des Gesamtvolumens der beiden Katalysatorbetten beträgt.

7. Verfahren nach Anspruch 4, dadurch gekennzeichnet, daß die Metalle des ersten Betts aus Palladium oder aus Gold bestehen.

8. Verfahren nach Anspruch 4, dadurch gekennzeichnet, daß die Metalle des ersten Betts aus Palladium und Platin bestehen.

9. Verfahren nach mindestens einem der Ansprüche 1 bis 8, dadurch gekennzeichnet, daß der bezüglich der beiden Katalysatorbetten eingesetzte Wasserstoff 100 bis 10000 ppm an $H_2S$ enthält.

10. Verfahren nach mindestens einem der Ansprüche 1 bis 9, dadurch gekennzeichnet, daß der Kohlenwasserstoffschnitt zwischen 5 und 50 ppm an Schwefel enthält.

## Claims

1. Process for isomerizing 1-butene into 2-butenes in a $C_4$ hydrocarbon cut containing butadienne and sulfur compounds, with hydrogenation of the butadienne, comprising :

(a) feeding said cut through a first catalyst bed comprising palladium and at least one additional metal selected from the group consisting of gold and platinum, these metals beign deposited on a support, in the presence of hydrogen, at a temperature ranging from 20 to 150°C, under a pressure from 1 to 50 atm.

(b) feeding at least the greater part of the effluent of the first catalyst bed through a second catalyst bed containing palladium deposited on alumina or silica, in the presence of hydrogen, at a temperature ranging from 20 to 150°C, under a pressure from 1 to 50 atm.

2. A process according to Claim 1, wherein both catalyst beds are placed in the same reaction zone, the temperature of the reaction zone ranging form 60 to 100°C and the pressure being 5-30 atm.

3. A process according to Claim 2, wherein the additional metal of the second catalyst bed is palladium and said metal has been introduced on the support in the form of a nitrate.

4. A process according to Claim 2, wherein the additional metal of the second catalyst bed is palladium and said metal has been introduced on the support in the form of a chloride.

5. A process according to Claim 1, wherein the support of the catalysts of both beds is an alumina with a specific surface smaller than 100 $m^2/g$.

6. A process according to Claim 2, wherein the amount of catalyst of the first bed represents, by volume, 10 to 70% of the total volume of both catalyst beds.

7. A process according to Claim 4, wherein the metals of the first bed are palladium and gold.

8. A process according to Claim 4, wherein the metals of the first bed are palladium and platinium.

9. A process according to Claim 1, wherein the hydrogen used on each catalyst bed contains 100 to 10000

ppm of H$_2$S.

10. A process accordin to Claim 1, wherein the hydrocarbons cut contains from 5 to 50 ppm of sulfur.